(19) Europäisches Patentamt
European Patent Office
Office européen des brevets

(11) Numéro de publication: **0 013 651**
**B1**

# (12) FASCICULE DE BREVET EUROPEEN

(45) Date de publication du fascicule du brevet: **15.06.83**

(51) Int. Cl.³: **C 07 H 15/04,** A 61 K 37/02, A 61 K 39/02

(21) Numéro de dépôt: **80400041.2**

(22) Date de dépôt: **11.01.80**

(54) **Muramyl-peptides fixés sur polymères peptidiques et médicaments les contenant.**

(30) Priorité: **12.01.79 FR 7900819**

(43) Date de publication de la demande: **23.07.80 Bulletin 80/15**

(45) Mention de la délivrance du brevet: **15.06.83 Bulletin 83/24**

(84) Etats contractants désignés: **AT BE CH DE FR GB IT LU NL SE**

(56) Documents cités:
**EP-A-0 003 833**
**DE-A-1 915 970**
**DE-A-2 450 355**

(73) Titulaire: **ANVAR Agence Nationale de Valorisation de la Recherche, 13, rue Madeleine Michelis, F-92522 Neuilly-sur-Seine (FR)**

(72) Inventeur: **Lefrancier, Pierre, 20, rue des Bleuets, F-91440 Bures sur Yvette (FR)**
Inventeur: **Parant, Monique, 33, rue Croulebarbe, F-75013 Paris (FR)**
Inventeur: **Audibert, Françoise, 44, rue Ibry, F-92200 Neuilly sur Seine (FR)**
Inventeur: **Chedid, Louis, 16-18, rue Gaston de Caillavet, F-75015 Paris (FR)**
Inventeur: **Choay, Jean, 130, Faubourg Saint-Honoré, F-75008 Paris (FR)**
Inventeur: **Sela, Michael, The Weizmann Institute of Science, Rehovot (IL)**
Inventeur: **Lederer, Edgar, 9 Boulevard Colbert, F-92330 Sceaux (FR)**

(74) Mandataire: **Gutmann, Ernest et al, Cabinet Plasseraud 84, rue d'Amsterdam, F-75009 Paris (FR)**

EP 0 013 651 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

BUNDESDRUCKEREI BERLIN

Muramyl-peptides fixés sur polymères peptidiques et médicaments les contenant

L'invention est relative à de nouveaux composés doués de propriétés biologiques et pharmacologiques de grande valeur, et notamment, parmi ces produits à ceux qui présentent des propriétés régulatrices des mécanismes immunitaires.

L'invention concerne aussi les applications de ces nouveaux produits, ainsi que les compositions spécialement appropriées à leur mise en oeuvre.

On sait que des efforts considérables sont consacrés depuis longtemps à la recherche d'agents doués de propriétés régulatrices des réactions immunitaires. Ces recherches ont conduit tout d'abord à la préparation de produits naturels extraits notamment de fragments de peptidoglycane des parois de mycobactéries, puis à des produits synthétiques à molécules relativement petites. On dispose ainsi de séries de produits aux propriétés très intéressantes.

Au cours de ces recherches en même temps que ces nouveaux produits, sont apparues de nouvelles propriétés et, par là même, de nouvelles possibilités d'utilisation. En retour, la nécessité s'est fait sentir de trouver des produits répondant au mieux aux applications découlant de la mise en oeuvre de ces propriétés nouvelles, soit qu'il s'agisse de favoriser au maximum un ou plusieurs types d'activité, soit que l'on souhaite scinder les différentes activités possibles pour disposer de produits à action très spécifique, soit encore que l'on veuille réduire ou supprimer certains effets secondaires indésirables.

Pour parvenir à ces objectifs, on s'est efforcé de modifier certaines formes d'administration, mais surtout des recherches ont été entreprises pour modifier l'activité des composés en question en faisant varier certains éléments de leur structure. Aussi est-il toujours de grande importance de pouvoir disposer de produits nouveaux permettant de diversifier les combinaisons de pripriétés disponibles dans le domaine de l'immunorégulation et, par suite, d'élargir le choix de l'utilisateur et l'adapter au mieux à l'utilisation projetée.

Parmi les modifications d'activité recherchées, l'une concerne l'allongement de la durée d'activité des produits administrés en réduisant leur vitesse d'élimination. La demanderesse a en effet constaté que les produits comme la N-acétyl-muramyl-L-alanyl-D-isoglutamine sont éliminés relativement rapidement, peut-être en raison de leur faible poids moléculaire.

Une autre modification souhaitée est éventuellement de pouvoir diriger le produit immunorégulateur vers des sites récepteurs déterminés de l'hôte auquel il est administré, conduisant ainsi notamment à une meilleure efficacité pour une dose administrée moindre.

Un autre but de l'invention est de permettre éventuellement la mise en évidence de nouvelles propriétés pharmacologiques ou combinaisons de ces propriétés. Dans cette optique, il était particulièrement souhaitable de disposer de produits dont l'indice thérapeutique, c'est-à-dire le rapport des doses effic aces aux doses limites pour lesquelles des phénomènes indésirables commencent à apparaître, soit aussi grand que possible.

Les améliorations et modifications souhaitées ont été atteintes, au moins en partie, par les produits faisant l'objet de la présente invention. Ces produits comprennent des polymèreres de nature peptidique dont une chaîne, dite ci-après chaîne principale, est formée de maillons correspondant de préférence à l'un au moins des acides aminés suivants, de préférence de la série L : l'acide $\alpha,\gamma$-diamino-butyrique, l'ornithine, la lysine et l'homo-lysine. Sur la chaine peptidique ainsi définie peuvent se brancher des chaînes également de nature peptidique dont les maillons correspondent à au moins l'un des résidus des acides aminés suivants: l'alanine, la glycine, l'acide $\alpha$-aminobutyrique, la valine, la leucine, l'isoleucine et la proline. Sur la fonction amine de cette structure polymérique (non engagée sur celle-ci) sont fixés de façon covalente des groupes muramyl-peptide (ou de leurs homologues ou dérivés) dont les deux premiers résidus d'acides aminés liés au groupe muramyle sont les stéréo-isomères de la série D. On rappellera que le second résidu présente nécessairement la structure de l'acide glutamique. Ladite fixation de façon covalente met en jeu l'une des fonctions carboxyle, de préference en $\gamma$ du résidu glutamique. Le polymère portant ces groupes muramyl-peptide a une masse moléculaire moyenne qui peut notamment varier entre 50 000 et 250 000.

De préférence, le rapport total du nombre de résidus aminoacyle contenus dans les ramifications vis-à-vis du nombre de résidus aminoacyle contenus dans la chaîne principale est de 1/1 à 1/30, notamment de 1/10 à 1/30.

Des polymères peptidiques du type de ceux qui font l'objet de la présente invention mais ne renfermant pas de groupements muramyl-peptide ont été décrits antérieurement. Ces polymères ont été étudiés en tant que porteurs d'antigènes synthétiques.

Par ailleurs, des composés glycopeptidiques du type muramyl-peptide dans lesquels les deux premiers acides aminés sont de la série D ont été décrits antérieurement, notamment dans la demande de brevet français n° 75 30948. Contrairement à ce que l'on connaissait des propriétés de la N-acétyl-muramyl-L-alanyl-D-isoglutamine, il était montré, dans cette demande de brevet, que le stéréo-isomère correspondant, dans lequel les deux acides aminés sont de la série D, ne présentait aucune propriété immunostimulante spécifique. Ce même composé ne stimule pas non plus l'immunité non spécifique, comme cela est indiqué par CHEDID et al., Proc. Nat. Acad. Sci. N.Y., 1977, 74, 2089. Autrement dit, ce composé ne favorisait pas la production d'anticorps contre l'antigène

administré conjointement.

A s'en tenir à ces éléments de la technique, il est certain que rien n'aurait suggéré de chercher à utiliser les muramyl-peptides en question sous quelque form que ce soit pour obtenir des propriétés stimulantes de l'immunité spécifique ou non spécifique. En particulier, il était tout à fait impossible de prévoir que la fixation d'un produit comme la N-acétyl-muramyl-D-alanyl-D-isoglutamine fixée sur le polymère peptidique présenterait une activité anti-infectieuse remarquable, comme on le verra dans les exemples d'essais pharmacologiques figurant dans cette demande. Contrairement à ce que les connaissances antérieures auraient pu laisser supposer, la demanderesse a découvert que les produits muramyl-peptide dérivés ou analogues du Mur-NAc-Ala-D-isoglutamine, lorsqu'ils sont fixés sur un support polymérique de nature peptidique du type en question, peuvent voir leur activité modifiée de façon avantageuse.

De préférence, pour faciliter l'utilisation pharmaceutique des composés selon l'invention, on choisit ceux dont la structure est telle qu'ils puissent être métabolisés par l'organisme de l'hôte auquel ils sont administrés. Dans ce sens, il est préférable qu'une partie au moins des résidus aminoacyle constituant le polymère peptidique soient de la série L.

Il est avantageux, bien que cela ne soit pas indispensable, que les composés selon l'invention soient solubles dans l'eau. Pour cette raison, il est avantageux d'utiliser un polymère présentant des ramifications et dont ces ramificaions soient formées de maillons aminoacyle appartenant à la fois aux séries L et D. Il semble en effet que la présence simultanée de ces deux formes, en rompant la régularité de structure des chaînes ramifiées, favorise le caractère hydrophile de ce polymère. Il est avantageux d'utiliser pour l fabrication de ces charges un mélange racémique de L-alanine et de D-alanine. Bien entendu, l'utilisation pour la formation de ces mêmes chaînes de proportions initiales inégales des deux isomères optiques, n'échapperait pas à la portée de l'invention.

Des polymères peptidiques préférés pour la présente invention sont ceux dont la chaîne peptidique principale est formée de L-lysine polymère.

De la même façon, des polymères peptidiques préférés sont ceux dont les chaînes en ramifications sont constituées par des résidus alanyle des séries D et L, et par des résidus prolyle de la série L.

De préférence, tout particulièrement, le polymère selon l'invention présente une chaîne principale constituée par la polymérisation de résidus L-lysyle sur laquelle les ramifications sont constituées par des chaînes polymérisées comportant les maillons D—L alanyle. De préférence, le polymère peptidique présente une masse moléculaire moyenne d'environ 80 000 et le rapport du nombre total de résidus L-lysine de la chaîne aux résidus D—L alanine des ramifications est compris entre 1/10 et 1/30.

Avantageusement, les groupes muramyl-peptide fixés sur les polymères peptidiques sont tels que la structure des composés selon l'invention répondent à la formule générale suivante:

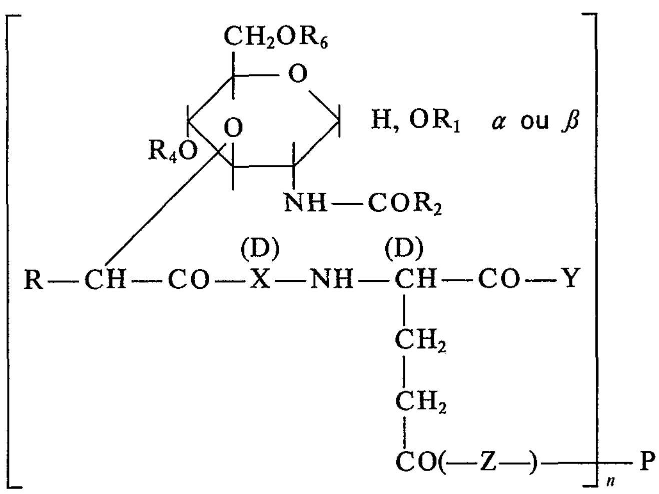

(I)

dans laquelle P représente la susdite structure polymère, éventuellement ramifiée, telle que définie ci-dessus et les substituants R, $R_1$, $R_2$, $R_4$, $R_6$, X, Y et Z ont les significations suivantes:

- R est soit un atome hydrogène, soit un groupe alcoyle comprenant de 1 à 4 atomes de carbone,
- $R_1$ est un atome d'hydrogène ou un groupe alcoyle ayant au plus 4 atomes de carbone, ou un groupe aryle ou alcoyl-aryle contenant au plus 10 atomes de carbone,
- $R_2$ est un atome d'hydrogène ou un groupe alcoyle, aryle ou alcoyl-aryle comportant au plus 22 atomes de carbone,
- $R_4$ est un atome d'hydrogène ou un radical acyle comprenant au plus 4 atomes de carbone,
- $R_6$ est un atome d'hydrogène, un groupe acyle saturé ou non, éventuellement ramifié, contenant de 1 à environ 90 atomes de carbone et pouvant, en outre, porter des groupes fonctionnels: hydroxyle, carboxyle, amino, cyclopropane, méthoxyle,

– X est un aminoacyle de série (D) du groupe comprenant: alanyle, arginyle, asparagyle, aspartyle, cystéinyle, glutaminyle, glutamyle, histidyle, hydroxyprolyle, isoleucyle, leucyle, lysyle, méthionyle, ornithyle, phénylalanyle, prolyle, séryle, thréonyle, tryptophanyle, tyrosyle et valyle,

– Y est soit $-OH$, soit un radical alcoxy comprenant de 1 à 10 atomes de carbone, soit un groupe $-NH_2$, les hydrogènes du groupe amino pouvant être substitués par des restes alcoyle comprenant de 1 à 10 atomes de carbone,

– Z peut être présent ou non dans la formule et représente de 1 à 3 résidus L ou D aminoacides identiques ou différents, du groupe de ceux indiqués pour X ou glycyle, et

– n est au plus égal au nombre de fonctions amines protées par la structure polymérique de P (notamment au nombre n–1 du groupe lysyle, lorsque la chaîne principale est une polylysine).

On notera que les homologues de l'acide muramique, au sens où cette expression a été utilisée plus haut, sont les composés qui se distinguent entre eux par des significations différentes des groupes R, $R_1$, $R_2$, $R_4$, $R_6$ et X, tels qu'ils apparaissent dans la formule II et que de même, l'expression »dérivés« concerne les composés qui se distinguent entre eux au niveau des significations des groupes Y et Z.

Dans cette formule, le deuxième groupe aminoacyle de la chaîne peptidique liée au groupe de type muramyle est le résidu D-glutamyle. Le premier groupe aminoacyle (désigné par X) est choisi parmi désigné les différents résidus aminoacyles de série (D) mentionnés ci-dessus. Parmi les composés de formule I, on préfère ceux dans lesquels le premier groupe aminoacyle est D-alanyle. Un deuxième type de composés préférés cet celui dans lequel cet aminoacyle est D-séryle.

Sont également avantageux les composés dans lesquels le premier groupe aminoacyle est D-prolyle, D-thréonyle ou D-valyle.

Entre le résidu D-glutamyle et la chaîne polymère peptidique peuvent s'intercaler un ou plusieurs résidus aminoacyles supplémentaires désignés dans la formule générale par Z.

De préférence, ces aminoacyles sont choisis parmi le groupe comprenant: alanyle, leucyle, glycyle, valyle et isoleucyle.

Le nombre d'aminoacyles entre le D-glutamyle et la chaîne P peut varier de 0 à 3, de préférence cependant il est soit 0, soit 1, soit 2.

En position $\alpha$ du résidu D-glutamyle, les variantes ou substitutions possibles sont représentées par Y. Y peut tout d'abord être un radical $-NH_2$, l'un au moins des atomes d'hydrogène du groupe amino pouvant être substitué par des restes alcoyle courts comprenant de 1 à 10 atomes de carbone. Il peut encore s'agir des formes estérifiées de l'acide Y étant alors un alcoxy comprenant de 1 à 10 atomes de carbone.

Dans une forme préférée, Y est $-NH_2$.

D'autres formes préférées sont constituées par les cas ou Y est soit $-OCH_3$, soit $OC_4H_9$, soit $OC_{10}H_{21}$.

Dans la forme préférée la plus usuelle, c'est-à-dire celle pour laquelle on retrouve la structure de l'acide muramique, R est $-CH_3$. Dans une autre forme préférée, le groupe R est un hydrogène; on retrouve alors la structure de l'homologue inférieur désigné sous le nom d'acide normuramique. Enfin, dans une autre forme préférée, R est $-C_2H_5$; à cette forme correspond la structure dite homomuramique.

L'hydroxyle porté par le carbone anomère dans la partie saccharidique des produits selon l'invention peut se présenter sous forme $\alpha$ ou $\beta$. Ce résidu osidique peut recevoir également différents substituants dont la littérature antérieure, relative aux agents adjuvants du type muramyl-peptide, a donné un certain nombre d'exemples. En particulier, la littérature décrit des produits dont les groupes fonctionnels du résidu osidique sont substitués par formation de groupements esters ou éthers pour les hydroxyles, ou par des groupements amides pour le radical amino en position 2.

Dans la formule générale des produits selon l'invention, les substituants du cycle glucopyranoside ont été désignés par $R_1$, $R_2$, $R_4$ et $R_6$. Les différentes positions ne présentent pas les mêmes possibilités de substitution, la position 6 étant celle pour laquelle la plus grande latitude est offerte.

Des composés préférés sont ceux dans lesquels un on plusieurs des substituants $R_1$, $R_4$ et $R_6$, indépendamment les uns des autres ou simultanément, sont l'hydrogène.

Des composés avantageux sont également ceux pour lesquels $R_4$ est le groupe acétyle.

Des composés préférés sont aussi ceux pour lesquels $R_6$ est un radical acyle contenant de 1 à 4 atomes de carbone, et notamment les radicaux acétyle ($COCH_3$), ou encore ceux pour lesquels $R_6$ est le groupe mycoloyle (environ $C_{80}$ à $C_{90}$) ou corynomycoloyle ($C_{32}$).

Des substituants $R_2$ préférés sont constitués par les groupes alcoyle comprenant de 1 à 4 atomes de carbone, et, de façon particulièrement préférée, $R_2$ est $CH_3$.

Parmi les composés selon l'invention sont particulièrement préférés ceux pour lesquels $R_1$, $R_4$, $R_6$ sont simultanément un atome d'hydrogène, R et $R_2$ sont $-CH_3$, X est D-alanyle, Y est $-NH_2$.

Les modes de préparation des muramyl-peptides ont été décrites dans la littérature pour un certain nombre d'entre eux. A toutes fins utiles, des modes généraux de préparation pour ces produits sont indiqués ci-après. Bien entendu, ces modes ne sont pas les seuls envisageables, et de nombreuses variantes peuvent également être utilisés.

Les produits selon l'invention sont préparés par synthèse ou, le cas échéant, par hémosynthèse. Dans un premier temps, le dérivé glycopeptidique est synthétisé puis dans un second temps, il est fixé de façon covalente sur la chaîne peptidique polymère en utilisant l'une ou l'autre des méthodes de couplage connues en la matière.

Pour aboutir à un composé glycopeptidique, diverses voies sont possibles. Dans tous les cas, la synthèse comporte une série d'étapes au cours desquelles les différents »fragments« constituant la structure d'ensemble des composés selon l'invention sont progressivement assemblés. Les principales différences entre les voies possibles se situent dans la séquence choisie pour l'assemblage des fragments. Les modes de réaction conduisant à la fixation d'un fragment aux fragments contigus (ou à des fragments pré-assemblés) sont dans l'ensemble peu modifiés par l'ordre dans lequel cette synthèse est conduite, à ceci près bien entendu que de cet ordre dépend, d'une part, le choix des groupes fonctionnels qui réagissent et qui, par conséquent, doivent être libres pour l'étape considérée, et, d'autre part, le choix des groupes qui doivent être bloqués pour ne pas intervenir au cours de cette même étape.

Le procédé de fabrication selon l'invention des produits nouveaux susdits est notamment caractérisé en ce que l'on réalise un couplage de façon covalente dans des conditions connues dans le domaine des synthèses peptidiques entre:

- d'une part, les fonctions amine libres d'une structure polymère de nature peptidique comportant une chaîne principale formée + partir d'unités aminoacyle correspondant de préférence à l'un au moins des acides aminés suivants: acide $\alpha$, $\gamma$-diamino-butyrique, ornithine, lysine et homolysine, et le cas échéant des ramifications elles-mêmes de nature peptidique, branchées sur la susdite chaîne principale et cinstituées de résidus aminoacyles correspondant, de préférence, à au moins l'un des aminoacides suivants: alanine, glycine, acide $\alpha$-aminobutyrique, valine, leucine, isoleucine et proline, et
- d'autre part, l'une des fonctions carboxyliques, de préférence la fonction $\gamma$-carboxyle, de composés du type muramyl-peptide, ou de leurs homologues et dérivés, dans lesquels la chaîne peptidique comprend un premier aminoacide de la série D, fixé au groupe muramyle (ou dérivé de celui-ci) et le second aminoacyle est dérivé de l'acide D-glutamique, auquel appartient la susdite fonction $\gamma$-carboxyle intervenant au niveau de la réaction de couplage.

De préférence, les composés du type muramyl-peptide mis en oeuvre dans le procédé selon l'invention sont caractérisés par la structure:

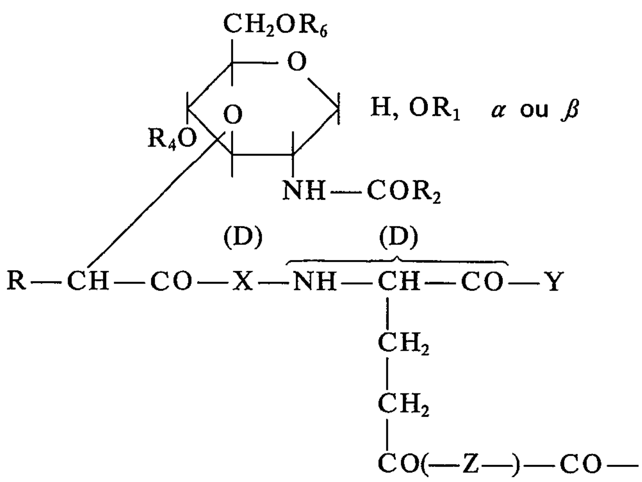

(II)

dans laquelle R, $R_1$, $R_2$, $R_4$, $R_6$, X, Y et Z ont les significations sus-indiquées.

En particulier, on fait agir un ester activé des composés du type muramyl-peptide, obtenu par action sur ceux-di d'un carbodiimide hydrosoluble et d'un alcool, notamment d'hydroxybenzotriazole au sein d'une solution dans un solvant non aqueux et miscible à l'eau, tel que le diméthylformamide, l'acétone ou le tétrahydrofurane, sur une solution aqueuse du polymère peptidique en solution aqueuse, à un pH sous lequel les fonctions amine libres du polymère peptidique sont libres et non salifiées, notamment à pH 8 – 9, de préférence à pH 8,5.

La réaction étant terminée, le produit est avantageusement obtenu par lyophilisation, redissous dans l'eau et ultrafiltré sur membrane ultrafiltrante permettant la séparation du polymère, d'une part, et de molécules de poids moléculaires plus faibles.

On a donné ci-après de façon succincte les principales indications relatives aux différentes opérations qui peuvent être mises en oeuvre pour synthétiser les produits selon l'invention, d'abord en envisageant séparément chaque étape, puis en indiquant quelques séquences types préférées.

a) Formation de l'acide muramique ou des analogues

Pour obtenir les analogues de l'acide N-acétyl-muramique de formule:

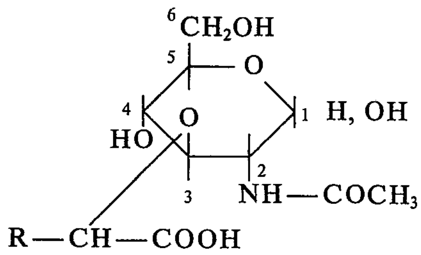

dans laquelle R a la signification précédemment indiquée, il est possible de partir d'un dérivé de la N-acétyl-2-déoxy-glucosamine dont les hydroxyles en position 1, 4 et 6 sont bloqués de façon traditionelle. Le mode de préparation d'un tel dérivé, le benzyl-2-acétamido-4,6-O-benzylidène-2-déoxy-D-glucopyranoside, est décrit notamment par P. H. GROSS et R. W. JEANLOZ (J. Org. Chem. 1967, 32, 2761).

La formation de l'acide N-acétyl-muramique ($R = CH_3$) ou d'un de ses analogues peut être réalisée de la manière décrite dans les demandes de brevets français n° 2 292 486 ou 2 355 505 (respectivement, pour ces demandes, $R = CH_3$ et $R = H$) reprenant la méthode décrite par OZAWA et JEANLOZ (J. Org. Chem., 1965, 30, 448).

Cette formation comprend par exemple la préparation d'un sel de sodium de l'hydroxyle en position 3 et la condensation ultérieure du dérivé sodé avec le sel ou l'ester d'un acide halogéné tel que les acides chloro-2-propionique ou chloroacétique pour reprendre le cas des deux demandes de brevets indiquées précédemment. Le composé halogéné utilisé de forme L peut être préparé suivant la méthode décrite par SINAY et al. (J. Biol. Chem., 1972, 247, 391). En utilisant les acides halogénés appropriés, il est possible de préparer tous les dérivés correspondant aux différentes significations de R. Ainsi, pour introduire un groupe R à 4 carbones, on peut utiliser les sels ou esters de l'acide chloro-2-butyrique.

Lorsqu'on utilise un ester d'acide halogéné, afin de pouvoir procéder à la condensation peptidique ultérieure, la fonction carboxylique peut être libérée par une hydrolyse appropriée.

b) Substitution sur le résidu saccharidique

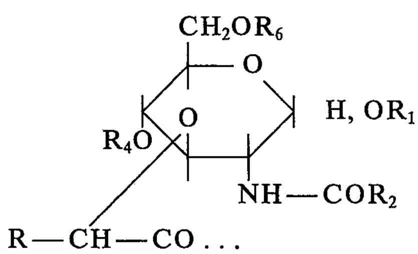

Partant des dérivés N-acétyl-muramiques bloqués dans les positions 1, 4, 6, comme obtenu en a, il est possible de préparer les différents composés analogues dans lesquels le groupe acétyl fixé sur l'azote en position 2 est remplacé par les substituants dont la nature est celle donnée dans la définition générale, c'est-à-dire un groupe alcoyle, aryle ou alcoyle-aryle éventuellement substitué et comportant au plus 22 atomes de carbone. Pour cette modification, on peut opérer de façon connue une hydrolyse de l'acétyle par une base forte, par exemple comme cela est décrit dans la publication de P. H. GROSS et JEANLOZ indiquée plus haut.

Le composé résultant, dans lequel un groupe amino est en position 2 du cycle glucopyranoside, peut alors être de nouveau soumis à un traitement d'acylation, dans des conditions traditionnelles, avec un agent acylant approprié correspondant au groupe $R_2$ que l'ont veut introduire. Comme agent acylant, on peut notamment utiliser les anhydrides ou les chlorures d'acides.

Les substitutions en position 1, 4 et 6 peuvent être réalisées par des méthodes qui ont été décrites antérieurement et qui sont traditionnelles dans la chimie des sucres. Lorsque les substituants envisagés sont différents les uns des autres, on procédera à autant de réactions de substitution successives qu'il y a de substituants distincts. Au cours de ces réactions, les positions ne devant pas être substituées ou celles qui doivent faire ultérieurement l'objet d'une autre substitution sont protégées temporairement par des groupes de blocage selon les modes habituels.

Les groupes de blocage initialement présents, dans le cas où l'on part, comme indiqué précédemment, de benzyl-2-acétamido-4,6-O-benzylidène-2-déoxy-D-glycopyranoside, sont éliminés

par exemple par action de l'acide acétique (à 60% 1 heure à reflux) et hydrogénation catalytique, comme décrit par exemple par MERSER et al. (Biochem. Biophys. Res. Commun., 1975, 66, 1316), ou par hydrogénation catalytique suivant la méthode de LEFRANCIER et al. (Int. J. Peptide Protein Res., 1977, 9, 249).

Les modes de substitution sont ceux traditionnellement utilisés. Pour obtenir les dérivés acylés, on opère à l'aide d'un agent acylant correspondant au substituant que l'on souhaite introduire (anhydride, chlorure d'acyle, etc).

Les positions 1, 4, 6 ne sont pas équivalentes pour ce qui concerne les réactivités. La position C-6 est la plus facile à substituer. Aussi, lorsque seule cette position doit être substituée, il est possible d'opérer sans bloquer les autres positions, avec une quantité d'agent de substitution équivalente à celle nécessaire pour la substitution d'une seule position.

Un exemple particulier de mode de préparation des dérivés substitués en position 6 est donné dans l'article de KUSUMOTO et al. (Tetrahedron Letters, 1976, 47, 4237).

Les substitutions sur le résidu osidique peuvent être effectuées avant ou après la fixation de la chaîne peptidique ou des fragments de celle-ci.

c) Chaîne peptidique

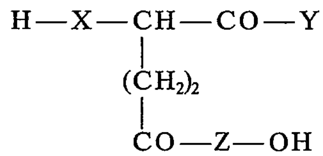

La fixation d'une chaîne peptidique sur l'acide N-acétyl-muramique, ou sur un analogue de celui-ci tels que deux qui ont été indiqués ci-dessus, est obtenue par des méthodes classiques dans le domaine de la synthèse des peptides. De telles méthodes ont été amplement décrites dans la littératures antérieure et en particulier dans les demandes de brevets français indiquées précédemment.

De façon générale, les synthèses glycopeptidiques peuvent être faites soit en fixant un premier aminoacide au groupe muramyle, puis en fixant au composé ainsi obtenu le second aminoacide, et ainsi de suite de proche en proche.Il est aussi possible de préparer séparément la chaîne peptidique entière aminoacide par aminoacide et de fixer celle-ci sur le groupe muramyle. Il est enfin possible de choisir des procédés intermédiaires dans lesquels on prépare des fragments de la chaîne, puis soit d'assembler ces fragments entre eux jusqu'à former la chaîne complète qui est ensuite fixée au groupe muramyle, soit de fixer un premier fragment au groupe muramyle, puis un second au produit ainsi obtenu, etc. Le choix de la séquence est guidé principalement par des raisons de commodité ou de rendement.

La substitution Y est aventageusement réalisée sur le groupe glutamyle avant la synthèse de la chaîne.

Les synthèses peptidiques sont réalisées suivant des méthodes classiques. A titre d'exemple, on peut utiliser les méthodes d'activation des carboxyles, comme la méthode dite des anhydrides mixtes. De façon avantageuse, la synthèse peptidique est réalisée à l'aide d'un composé du type carbodiimide tel que le N,N'-dicyclohexylcarbodiimide ou des carbodiimides équivalents. In trouvera une revue des modes de synthèse peptidique traditionnels dans J. H. JONES, Chemistry and Industry, 723 (1974). On peut aussi se reporter aux demandes de brevets français suivantes: 2 288 527, 2 343 482, 2 343 483, 2 343 484, 2 358 159, 2 369 292 et à l'article de LEFRANCIER et al. (Int. J. Peptide Protein Res., 1977, 9, 249).

La formation des dérivés estérifiés ou amidés correspondant au groupe Y est obtenue de façon connue. On peut en particulier se reporter aux demandes de brevets français indiquées ci-dessus, et notamment aux demandes 2 343 483, 2 343 484, 2 358 159 et 2 369 292.

Le schéma représente des séquences réactionnelles conduisant à l'obtention des dérivés glycopeptidiques qui seront ensuite couplés sur la chaîne polymère peptidique. On part d'un dérivé (1) avec $R_1$ radical benzyle, décrit par GROSS et JEANLOZ (J. Org. Chem., 1967, 32, 2759). Pour obtenir le même composé dans lequel $R_1$ est un groupement alcoyle ou aryle-alcoyle, on peut utiliser la méthode de préparation des $\alpha$- ou $\beta$-glycosides correspondants également décrite dans ce même article, ou toute méthode connue pour de telles préparations en chimie des oligosaccharides.

Si l'on veut modifier la nature du groupe N-acyle, le groupe N-acétyle peut être hydrolysé comme décrit toujours par GROSS et JEANLOZ, pour aboutir aux dérivés de formule (2). Les dérivés (2) peuvent être ensuite N-acylés sélectivement par action d'anhydride d'acides carboxyliques pour aboutir aux dérivés de formule (3). Les dérivés de formule (4) peuvent être obtenus à partir des précédents suivant la méthode décrite par OZAWA et JEANLOZ (J. Org. Chem. 1965, 30, 448), au moyen d'un acide L-$\alpha$-chloroalcanoïque.

Les dérivés de formule (4) sont couplés avec un dérivé peptidique de formule générale

H – X – D – Glu – (Z – OBzl) – OXY

chlorhydrate. Formule dans laquelle X correspond à un acide aminé, et Y par exemple à un radical amino-, méthyl-amino-, méthoxy- ou glycyl-amide. Ces divers dérivés peptidiques sont préparés suivant les méthodes décrites par LEFRANCIER et al. (Int. Peptide Protein Res., 1977, 9, 249, et Int. J. Peptide Protein Res., 1978). Les méthodes de couplage utilisées pour obtenir des dérivés glycopeptidiques de formule (5) sont également décrites dans les articles précédemment cités. Toutefois, aussi bien dans la synthèse des dérivés dipeptidiques que dans celle des dérivés de la formule (5), toute méthode de couplage utilisée en synthèse peptidique peut être utilisée.

L'hydrogénation catalytique des composés de formule (5) est effectuée de façon classique (LEFRANCIER et. al., Int. J. Peptide Protein Res., 1977, 9, 249) pour aboutir aux composés de formule (6).

Les dérivés de formule (6) sint couplés, par exemple suivant la méthode décrite plus loin en détail, au moyen d'un carbodiimide hydrosoluble et de l'hydroxybenzotriazole, avec la chaîne polymère peptidique. On obtient les composés de formule (7).

Dans une variante, les dérivés de formule (5) subissent une débenzylidénation sélective telle que décrite par MERSER et al. (Biochem. Biophys. Res. Commun., 1975, 66, 1316) pour donner les dérivés de formule (8). L'acylation sélective de l'hydroxyle primaire en position 6 du résidu saccharidique peut alors se faire directement par action d'un faible excès d'anhydride d'acides carboxyliques ou d'acyl-imidazole. On obtient des dérivés de formule (9).

Les Dérivés de la formule (9) peuvent être synthétisés suivant une séquence totalement différente (schéma II, formule 4) semblable à celle mise au point par KUSUMOTO et al. (Tetrahedron Letters, 1976, 47, 4237).

Après hydrogénation catalytique des composés (9), effectuée comme usuellement en présence de palladium 5% sur charbon, on obtient les composés de formule (10) auxquels, comme précédemment, peut être couplé un reste pour donner le composé (11) selon l'invention.

Dans une autre variante, les dérivés de formule (8) sont diacylés sur les deux hydroxyles en position 4 et 6 du résidu saccharidique par action d'un excès d'anhydride d'acide carboxylique, puis soumis à une hydrogénation catalytique effectuée comme usuellement en présence de palladium 5% sur charbon, pour obtenir les composés de formule (13). Après couplage avec la chaîne polymère peptidique comme précédemment, on obtient les composés (14) selon l'invention.

Schema (I)

Séquences de synthese des composés glycopeptidiques

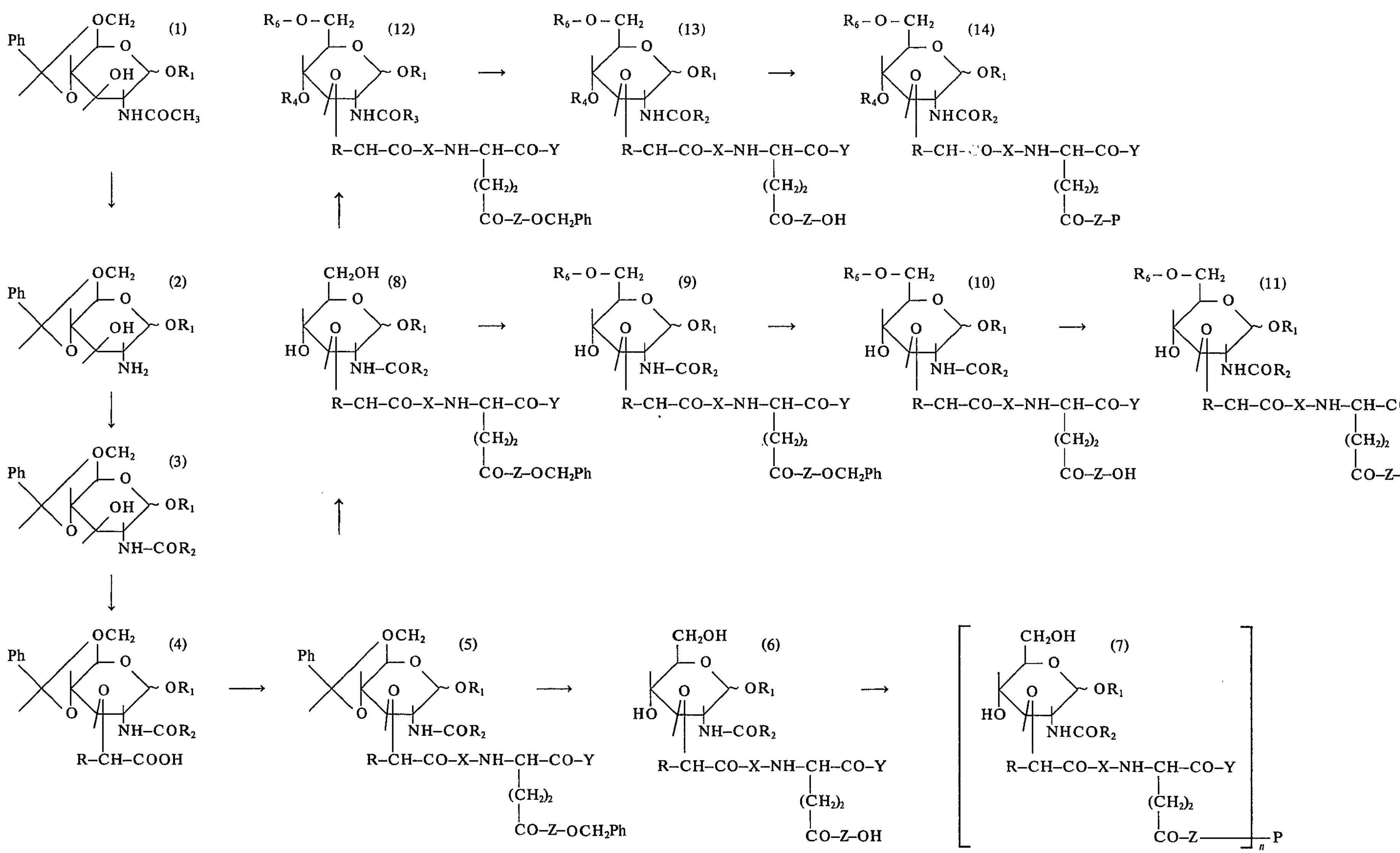

Schema (II)

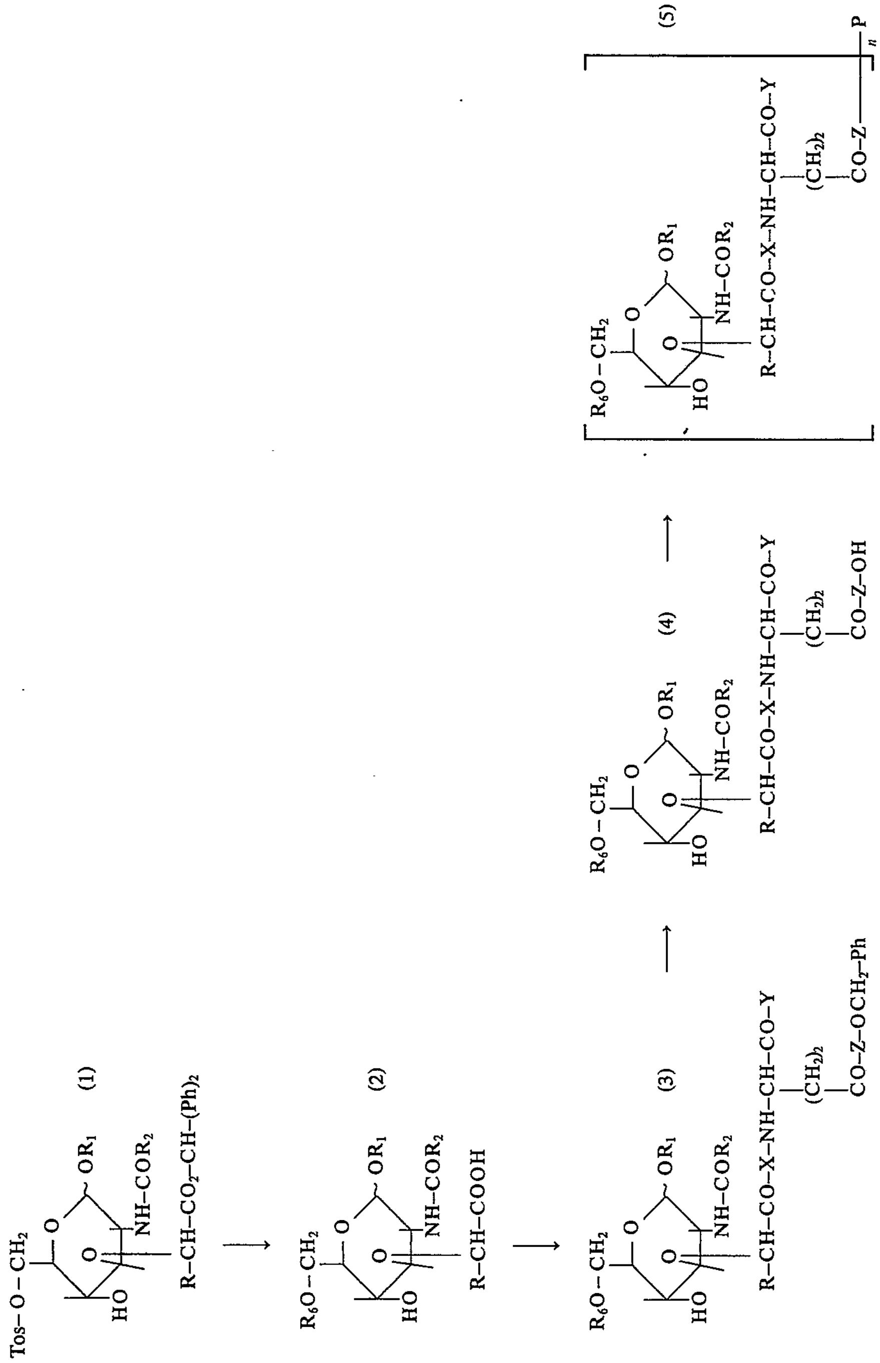

(1) (2) (3) (4) (5)

L'invention est aussi relative à des modes d'utilisation des composés répondant aux définitions précédentes, notamment en tant que réactif ou en tant que substance active dans des compositions pharmaceutiques.

L'invention concerne des réactifs biologiques qui peuvent être constitués à l'aide des composés selon l'invention, notamment en vue d'étudier les éventuelles propriétés adjuvantes standards ou, au contraire, comme agent susceptible de s'opposer à certains effets liés à l'administration de substances immunosuppressives.

Plus particulièrement, l'invention est relative à des médicaments renfermant à titre de principe actif au moins l'un des composés selon l'invention, ce médicament étant applicable en tant que régulateur de la réponse immunitaire du sujet auquel il est administré.

Ces médicaments sont notamment applicables pour le traitement des maladies infectieuses d'origine bactérienne ou parasitaire, ou l'inhibition d'affections tumorales. Particulièrement, les médicaments renfermant les composés selon l'invention peuvent être utilisés pour le traitement des infections causées par des agents résistant aux antibiotiques.

L'application de ces médicaments n'est pas seulement curative, elle peut aussi se faire à titre préventif. Les médicaments selon l'invention peuvent être administrés à l'hôte — animal ou être humain — de toute façon appropriée à l'obtention de l'effet désiré.

L'invention concerne naturellement aussi les diverses compositions pharmaceutiques auxquelles les composés selon l'invention peuvent être incorporés, le cas échéant en association avec d'autres substances actives.

Des compositions phar 1aceutiques avantageuses sont constituées par des solutions ou suspensions injectables contenant une dose d'au moins un produit selon l'invention. De préférence, ces solutions ou suspensions sont réalisées dans une phase aqueuse stérilisée isotonique, de préférence saline ou glucosée.

L'invention concerne plus particulièrement de telles suspensions ou solutions qui sont aptes à être administrées par injections intradermiques, intramusculaires ou sous-cutanées, ou encore par scarifications.

D'autres compositions pharmaceutiques avantageuses sont constituées par les formes liposomiques des composés selon l'invention. Comme on le sait, les liposomes, en raison de la nature lipidique (et notamment phospholipidique) des éléments entrant dans leur composition, constituent, pour certains cas, une présentation particulièrement appropriée.

L'invention concerne aussi des compositions pharmaceutiques administrables par d'autres voies, notamment par voie orale ou rectale, ou encore sous des formes destinées à venir en contact avec des muqueuses, notamment les muqueuses oculaires, nasales, pulmonaires ou vaginales.

En conséquence, elle concerne des compositions pharmaceutiques dans lesquelles l'un au moins des composés selon l'invention se trouve associé à des excipients pharmaceutiquement acceptables, solides ou liquides, adaptés à la constitution de forme orale, oculaire ou nasale, ou avec des excipients adaptés à la constitution de formes d'administration rectale, ou encore avec des axcipients adaptés à l'administration vaginale, par exemple gélatineux. Elle concerne enfin des compositions destinées à la voie pulmonaire, notamment des solutions préparées en vue de l'administration au moyen d'un appareil d'aérosol classique.

L'invention consiste également en un procédé visant à renforcer les défenses immunitaires de l'hôte, consistant à lui administrer une dose efficace de l'un au moins des produits selon l'invention, sous l'une des formes d'administration qui a été évoquée ci-dessus. A titre d'exemple de doses susceptibles d'induire une action, on mentionnera des doses de 10 à 1000 µg par kg de corps, par exemple de 50 µg, lorsque l'administration est effectuée par voie parentérale, ou encore d'une dose de 200 à 20 000 µg par kg de corps, par exemple de 1000 µg, pour d'autres modes d'administration, tels que par exemple la voie orale.

L'invention est décrite de façon plus détaillée dans les exemples qui suivant relatifs à la préparation d'un produit selon l'invention et de divers essais concernant les propriétés pharmacologiques de ce produit.

### Préparation du polymère peptidique support (A — L) ou multi-poly (DL-alanyl)-poly(L-lysine)

La préparation est conduite à partir d'anhydride de DL alanine et de poly-L-lysine.

On place dans un ballon d'un litre 1 gramme de bromhydrate de poly-L-lysine en solution dans 300 ml de tampon phosphate à 0,05 mole/litre correspondant à un pH de 7. Le ballon est refroidi à environ 2°C dans un bain de glace. On ajoute, sous agitation énergique, 14 g d'anhydride de DL-alanine en solution dans 200 ml de dioxane anhydre. On observe un fort dégagement de $CO_2$.

In maintient le ballon dans le bain de glace et sous agitation pendant une nuit. La solution récupérée est dialysée plusieurs fois contre de l'eau distillée pour éliminer les constituants de faible poids moléculaire, puis elle est lyophilisée.

Pour parachever la purification, il est possible également de chromatographier la solution après

dialyse sur une colonne d'un gel de filtration, par exemple un gel connu sous la désignation SEPHADEX.

Préparation de multi-[poly-(N-acétyl-muramyl-D-alanyl-D-isoglutaminyl)-D,L-alanyl]-poly-L-lysine, soit de façon abrégée le MDP (D-D) (A – L)

675 mg (1,35 mmole) de N-acétyl-muramyl-D-alanyl-D-isoglutamine sont dissous dans 12,5 ml de diméthylformamide. A cette solution sont successivement ajoutés 187,5 mg d'hydroxy-benzotriazole et 575 mg de N-éthyl-N'-(diméthylaminopropyl)-carbodiimide, chlorhydrate. Après 1 heure, ce mélange réactionnel est ajouté à une solution aqueuse (25 mg) à 250 mg du polymère peptidique précédemment préparé (correspondant à 135 μ équivalents de fonctions amines théoriquement disponibles) ajustée à pH 8,5 avec une solution molaire de $NaHCO_3$.

La réaction se poursuit pendant 24 heures, 80 ml d'eau sont alors ajoutés et le produit est obtenu après lyophilisation. Il est redissous dans 50 ml d'eau, ultrafiltré sur un filtre Amicon PM 10, et finalement obtenu après lyophilisation et dessication. On obtient ainsi 226 mg du produit selon l'invention.

Le produit obtenu est analysé de la façon suivante.

Une fraction aliquote (1 mg) est hydrolysée (HCl 6 N, 24 h à 110° en tube scellé sous vide), puis il est procédé à une analyse en acides aminés: acide muramique-380, acide glutamique-430, alanine-11 463, lysine-450 (proportions exprimées en monomoles).

Basé sur les résultats de l'acide glutamique et de la lysine, le rendement obtenu est de 95,5%, et le rapport molaire est MDP(D-D)Lys/Ala : 0,64/1/21, soit encore 0,211 mg de MDP(D-D) par mg de MDP(D-D)-AL.

## PROPRIETES PHARMACOLOGIQUES

Les résultats figurant dans la suite concernent un produit type selon l'invention. Systématiquement, dans ces essais, le produit selon l'invention est comparé au glycopeptide correspondant, non fixé sur le polymère peptidique. Pour faciliter la comparaison, on a indiqué les quantités de glycopeptide contenues dans les doses essayées de produits selon l'invention.

Pour simplifier les écritures, les abréviations suivantes sont utilisées:

| | |
|---|---|
| MDP(D-D) | N-acétyl-muramyl-D-alanyl-D-isoglutamine |
| (A – L) | polymère formé par une chaîne L-lysine avec des ramifications D-L-alanine |
| MDP(D-D)(A – L) | N-acétyl-muramyl-D-alanyl-D-isoglutamine fixé sur le polymère (A – L). |

### 1. Activité anti-infectieuse vis-à-vis de Klebsiella

Le protocole des essais est décrit dans l'article CHEDID L. et col., Proc. Natl. Acad. Sci. USA, 1977, 74, 2089.

On a ainsi établi de façon préalable un mode expérimental permettant de mettre en évidence le caractère anti-infectieux des produits. On a montré qu'une dose d'environ $10^4$ Klebsiella pneumoniae, injectée par voie intramusculaire à des souris, entraîne le décès progressif d'une part importante, sinon de la totalité, des animaux dans la semaine suivant l'inoculation. Après huit jours, la survie des animaux est définitivement acquise.

On a suivi la survie de groupes de souris inoculées dans les conditions indiquées ci-dessus et traitées à l'aide de produits selon l'invention.

Pour ces essais, on a utilisé des souris hybrides (C57B1/6 × AKR)F1 élevées à l'INSTITUT PASTEUR, à partir de souches provenant de l'élevage du C.N.R.S. à ORLEANS.

L'infection par Klebsiella pneumoniae, souche de type capsulaire 2, biotype d, est faite à partir d'une culture de 16 heures en milieu pour pneumocoques (n° 53 515, INSTITUT PASTEUR). La dose infectante est $1{,}5 \cdot 10^4$ Klebsiella; elle est administrée par voie intramusculaire.

L'administration du produit testé est réalisée par voie intraveineuse dans 0,2 ml du soluté physiologique apyrogène, les témoins recevant le soluté seul. Elle est réalisée 24 heures avant l'inoculation.

Les résultats de ces essais sont rapportés dans le tableau suivant. Le pourcentage de protection indiqué est la différence des pourcentages de survivants du groupe traité par rapport au groupe témoin.

| | Doses*) (μg) | Nombre de souris | Nombre de survivants au J + 3 | J + 5 | J + 8 | Pourcentage de protection |
|---|---|---|---|---|---|---|
| Témoins | – | 32 | 16 | 7 | 4 | – |
| MDP (D-D) | 100 | 30 | 21 | 12 | 7 | 6 |
| | 1000 | 16 | 6 | 4 | 4 | 9 |
| MDP (D-D) (A–L) | 1 | 24 | 15 | 7 | 6 | 9 |
| | 10 | 32 | 26 | 19 | 17 | 38 |

*) Doses exprimées en poids de glycopeptide.

Ces résultats font apparaître une profonde différence dans les propriétés du glycopeptide selon qu'il est ou non fixé sur le polymère. Même à fortes doses, le MDP(D-D) seul n'est pratiquement pas anti-infectieux, alors que l'activité anti-infectieuse du produit selon l'invention est très sensible même pour des doses relativement faibles.

Des essais analogues ont été réalisés sur des souriceaux de 7 jours qui présentent une très grande sensibilité à l'infection par Klebsiella, comme indiqué par M. PARANT et al. (Proc. Acad. Sci. N.Y., 1978, 75, 33–95).

Dans ces essais, le produit étudié est administré, par voie sous-cutanée, 24 heures avant l'épreuve infectante. Pour cette dernière, la dose infectante est de 1,2. $10^3$ Klebsiella; elle est administrée aussi par voie sous-cutanée.

| Traitement s. c. à J – 1 | Doses (μg) | Nombre des souris | Nombre de survivants au J + 3 | J + 5 | J + 8 |
|---|---|---|---|---|---|
| Témoins | – | 53 | 5 | 1 | 0 |
| (A–L) | 50 | 40 | 20 | 7 | 0 |
| MDP (D-D) | 10 | 10 | 0 | 0 | 0 |
| | 100 | 16 | 3 | 0 | 0 |
| MDP (D-D) (A–L) | 1 | 39 | 19 | 6 | 3 |
| | 10 | 75 | 50 | 28 | 18 |

Les résultats de ces essais, pour le produit selon l'invention, font apparaître une protection significative des animaux ainsi qu'un fort retard dans la mortalité chez les animaux qui ne sont pas définitivement protégés.

2. Essais de sensibilisation

Ces essais ont été entrepris pour étudier les éventuelles propriétés immunogènes des produits selon l'invention. Dans le même temps, des essais de sensibilisation vis-à-vis de divers produits sont réalisés.

Les essais sont effectués sur des cobayes. Les préparations figurant à la colonne 1 du tableau du résultats sont injectées par voie plantaire. Elles comprennent 100 µg du produit testé incorporé à l'adjuvant complet de Freund (ACF), constitué par une émulsion d'eau dans l'huile contenant des mycobactéries tuées dans la phase huileuse ou à l'adjuvant incomplet de Freund (AIF) constitué par une émulsion eau dans l'huile sans mycobactéries.

Trois semaines après cette première injection, on injecte par voie dermique 50 µg des produits indiqués en tête des colonnes de résultats. Les réactions éventuelles sont observées 48 heures plus tard.

| | MDP | MDP (D-D) | MDP (D-D) (A–L) | (A–L) | OV |
|---|---|---|---|---|---|
| AIF + MDP | – | – | – | – | |
| ACF + MDP | – | – | – | – | |
| AIF + MDP + OV | – | – | – | – | + |
| AIF + MDP (D-D) (A–L) | – | – | – | – | |
| ACF + MDP (D-D) (A–L) | – | – | – | – | |
| AIF + MDP (D-D) (A–L) + OV | – | – | – | – | – |

Ces résultats montrent notamment que le MDP(D-D) (A–L) ne déclenche pas de réaction de sensibilité ni vis-à-vis de lui-même, ni vis-à-vis d'agents adjuvants comme le MDP, et n'est pas adjuvant de l'immunité spécifique lorsqu'il est administré avec l'ovalbumine. La même observation a été faite pour le produit administré dans une solution isotonique saline avec l'albumine de sérum bovin.

3. Etude de la toxicité et de l'effet pyrogène

On a étudié la toxicité des produits par administration parentérale à des souris surrénalectomisées pour les ren rendre particulièrement sensibles aux endotoxines.

Par ailleurs, on a déterminé les doses pyrogènes chez le lapin, c'est-à-dire les doses provoquant une augmentation de température égale ou supérieure à 0,6° C (Pharmacopée Européenne, Vol. 2, 1971, pages 58–60).

On constate que l'indice thérapeutique est très amélioré. En effet, s'il y a un certain accroissement de toxicité, celui-ci n'est en rien comparable à l'accroissement de l'activité qui est très important.

**Revendications pour les états contractants: BE, CH, DE, FR, GB, IT, LU, NL, SE**

1. Produit comportant une structure polymérique de nature peptidique comportant une chaîne principale, formée de maillons aminoacyle correspondant de préférence à l'un au moins des acides aminés suivants, de préférence de la série L: l'acide $\alpha,\gamma$-diamino-butyrique, l'ornithine, la lysine et l'homo-lysine et, de préférence, branchées sur cette chaîne principale, des chaînes ou ramifications de nature peptidique dont les maillons correspondent de préférence à au moins l'un des résidus des acides aminés suivants: l'alanine, la glycine, l'acide $\alpha$-aminobutyrique, la valine, la leucine, l'isoleucine et la proline, les fonctions amine de cette structure polymérique (non engagée dans celle-ci) étant au moins en partie reliées de façon covalente à des groupes muramyl-peptide (ou de leurs homologues ou dérivés), chacun desquels comprenant un groupe peptide dont le résidu d'acide aminé lié au groupe muramyle est un stéréo-isomère de la série D, et le second résidu amine est dérivé de l'acide D-glutamique.

2. Produit selon la revendication 1, caractérisé par la formule:

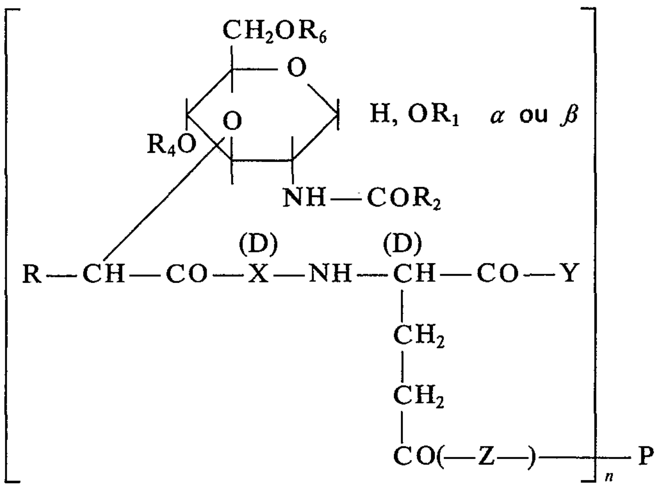

(I)

dans laquelle P représente la susdite structure polymère, éventuellement ramifiée, telle que définie ci-dessus et les substituants R, $R_1$, $R_2$, $R_4$, $R_6$, X, Y et Z ont les significations suivantes:

- R est soit un atome d'hydrogène, soit un groupe alcoyle comprenant de 1 à 4 atomes de carbone,
- $R_1$ est un atome d'hydrogène ou un groupe alcoyle ayant au plus 4 atomes de carbone, ou un groupe aryle ou alcoyl-aryle simple ou substitué contenant au plus 10 atomes de carbone,
- $R_2$ est un atome d'hydrogène ou un groupe alcoyle, aryle ou alcoyl-aryle éventuellement substitué et comportant au plus 22 atomes de carbone,
- $R_4$ est un atome d'hydrogène ou un radical acyle comprenant au plus 4 atomes de carbone,
- $R_6$ est un atome d'hydrogène, un groupe acyle saturé ou non, éventuellement ramifié, contenant de 1 à 90 atomes de carbone et pouvant, en outre, porter des groupes fonctionnels: hydroxyle, carboxyle, amino, cyclopropane, méthoxyle,
- X est un aminoacyle de série (D) du groupe comprenant: alanyle, arginyle, asparagyle, aspartyle, cystéinyle, glutaminyle, glutamyle, histidyle, hydroxyprolyle, isoleucyle, leucyle, lysyle, méthionyle, ornithyle, phénylalanyle, prolyle, séryle, thréonyle, tryptophanyle, tyrosyle et valyle,
- Y est soit $-OH$, soit un radical alcoxy comprenant de 1 à 10 atomes de carbone, soit un groupe $-NH_2$, les hydrogènes du groups amino pouvant être substitués par des restes alcoyle comprenant de 1 à 10 atomes de carbone,
- Z peut être présent ou non dans la formule et représente de 1 à 3 résidus L ou D aminoacides identiques ou différents, du groupe de ceux indiqués pour X ou glycyle, et
- n est au plus égal au nombre de fonctions amines portées par la structure polymérique de P.

3. Produit selon la revendication 1 ou 2, caractérisé en ce qu'il présente une masse moléculaire moyenne comprise entre 50 000 et 250 000, et qu'il est métabolisable.

4. Produit selon l'une quelconque des revendications précédentes, caractérisé en ce que la chaîne principale de la susdite structure polymère est formée, au moins en partie, de résidus L-lysyle.

5. Produit selon la revendication 4, caractérisé en ce que les ramifications de la susdite structure polymère sont constituées par des résidus alanyle.

6. Produit selon la revendication 5, caractérisé en ce que les résidus alanyle sont en partie les stéréo-isomères de la série D et en partie ceux de la série L.

7. Produit selon la revendication 5 ou la revendication 6, caractérisé en ce que le rapport du nombre de résidus aminoacides de la chaîne principale par rapport à celui des ramifications est de 1/1 à 1/30, de préférence de 1/10 à 1/30.

8. Produit selon l'une quelconque des revendications 1 à 7, caractérisé en ce que X est un résidu D-alanyle ou D-séryle.

9. Produit selon la revendication 8, caractérisé en ce que Y est $-NH_2$, $-OCH_3$, $-OC_4H_9$ ou $OC_{10}H_{21}$.

10. Produit selon l'une quelconque des revendications 1 à 9, caractérisé en ce que Z comprend 1 ou 2 résidus aminoacides choisis parmi ceux correspondant à l'alanine, la glycine, la valine, la leucine et l'isoleucine.

11. Produit selon l'une quelconque des revendications 1 à 10, caractérisé en ce que R est $-CH_3$.

12. Produit selon l'une quelconque des revendications 1 à 11, caractérisé en ce que $R_1$, $R_4$ et $R_6$ sont l'hydrogène et $R_2$ est $-CH_3$.

13. Produit selon l'une quelconque des revendications 1 à 8, caractérisé en ce que les motifs muramyle sont des N-acétyl-muramyl-D-alanyl-D-isoglutaminyle.

14. Composition pharmaceutique, caractérisée en ce qu'elle contient une dose d'au moins un produit

selon l'une quelconque des revendications 1 à 13 en association avec des véhicules ou excipients pharmaceutiquement acceptables.

15. Composition pharmaceutique selon la revendication 14, constituée par une solution ou suspension du ou des produits dans un milieu injectable.

16. Composition pharmaceutique selon la revendication 15, caractérisée en ce que le milieu injectable est constitué par une solution aqueuse pour injection, notamment une solution isotonique saline ou glucosée et stérile.

17. Composition pharmaceutique selon la revendication 14, caractérisée en ce que le produit est associé à des excipients respectivement adaptés à l'administration orale, à l'application sur les muqueuses oculaires, des voies respiratoires ou vaginales, ou l'administration rectale.

18. Composition pharmaceutique selon la revendication 14, caractérisée en ce qu'elle est présentée sous forme de liposome.

19. Composition pharmaceutique selon la revendication 14, caractérisée en ce que, pour un traitement antiinfectieux et une administration parentérale, les doses unitaires contiennent de 10 à 1000 µg du susdit produit.

20. Composition pharmaceutique selon la revendication 14, caractérisée en ce que, pour un traitement antiinfectieux et une administration orale, les doses unitaires contiennent de 200 à 20 000 µg du susdit produit.

**Revendications pour l'etat contractant: AT**

1. Procédé de fabrication d'un produit comportant une structure polymérique de nature peptidique contenant une chaîne principale formée à partir d'unités aminoacyle et, le cas échéant, branchées sur cette chaîne principale, des chaînes ou ramifications également de nature peptidique, ladite structure peptidique comportant en outre des fonctions amine non engagées dans ladite structure polymère, mais au moins en partie reliées de façon covalente à des groupes muramyl-peptide (ou de leurs homologues ou dérivés) chacun desquels comprenant un groupe peptide dont le résidu d'acide aminé lié au groupe muramyle est un stéréo-isomère dela série D, et le second résidu amine est dérivé de l'acide D-glutamique, caractérisé en ce que l'on réalise und couplage de façon covalente dans des conditions connues dans le domaine des synthèses peptidiques entre:

- d'une part, des fonctions amine libres d'une structure polymère de nature peptidique comportant une chaîne principale formée à partir d'unités aminoacyle correspondant de préférence à l'un au moins des acides aminés suivants: acide $\alpha$, $\gamma$-diamino-butyrique, ornithine, lysine et homolysine, et le cas échéant des ramifications elles-mêmes de nature peptidique, branchées sur la susdite chaîne principale et constituées de résidus aminoacyles correspondant, de préférence, à au moins l'un des aminoacides suivants: alanine, glycine, acide $\alpha$-aminobutyrique, valine, leucine, isoleucine et proline, et
- d'autre part, un ester activé, en particulier d'un composé du type muramyl-peptide présentant la structure définie par la formule

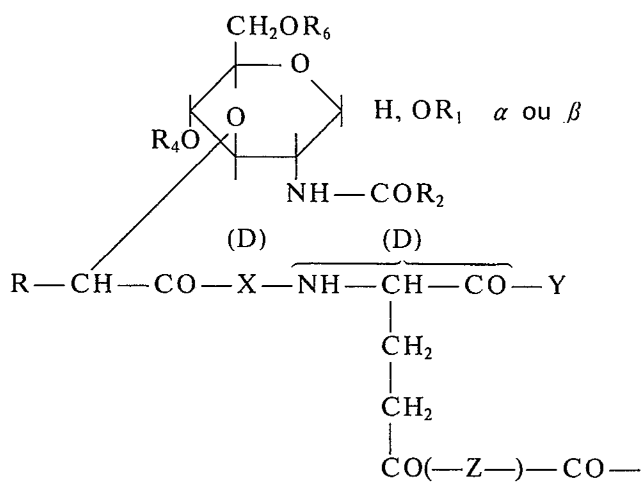

(II)

dans laquelle:

- R est soit un atome d'hydrogène, soit un groupe alcoyle comprenant de 1 à 4 atomes, de carbone,
- $R_1$ est un atome d'hydrogène ou un groupe alcoyle ayant au plus 4 atomes de carbone, ou un groupe aryle ou alcoyl-aryle simple ou substitué contenant au plus 10 atomes de carbone,

- $R_2$ est un atome d'hydrogène ou un groupe alcoyle, aryle ou alcoyl-aryle éventuellement substitué et comportant au plus 22 atomes de carbone,
- $R_4$ est un atome d'hydrogène ou un radical acyle comprenant au plus 4 atomes de carbone,
- $R_6$ est un atome d'hydrogène, un groupe acyle saturé ou non, éventuellement ramifié, contenant de 1 à 90 atomes de carbone et pouvant, en outre, porter des groupes fonctionnels: hydroxyle, carboxyle, amino, cyclopropane, méthoxyle,
- X est un aminoacyle de série (D) du groupe comprenant: alanyle, arginyle, asparagyle, aspartyle, cystéinyle, glutaminyle, glutamyle, histidyle, hydroxyprolyle, isoleucyle, leucyle, lysyle, méthionyle, ornithyle, phénylalanyle, prolyle, séryle, thréonyle, tryptophanyle, tyrosyle et valyle,
- Y est soit $-OH$, soit un radical alcoxy comprenant de 1 à 10 atomes de carbone, soit un groupe $-NH_2$, les hydrogènes du groupe amino pouvant être substitués par des restes alcoyle comprenant de 1 à 10 atomes de carbone,
- Z peut être présent ou non dans la formule et représente de 1 à 3 résidus L ou D aminoacides indentiques ou différents, du groupe de ceux indiqués pour X ou glycyle.

2. Procédé selon la revendication 1, caractérisé en ce que l'ester activé du composé de type muramyl-peptide a été obtenu par action sur un composé de muramyl-peptide correspondant d'un carbodiimide hydrosoluble et d'un alcool, et que la réaction est effectuée au sein d'une solution aqueuse à pH 8 – 9.

3. Procédé selon la revend ation 1 ou la revendication 2, caractérisé en ce que la structure polymère est métabolisable et en ce que le produit final obtenu présente une masse moléculaire moyenne comprise entre 50 000 et 250 000.

4. Procédé selon l'une quelconque des revendications 1 à 3, caractérisé en ce que la chaîne principale de la susdite structure polymère est formée, au moins en partie, de résidus L-lysyle.

5. Procédé selon la revendication 4, caractérisé en ce que les ramifications de la susdite structure polymère sont constituées par des résidus alanyle.

6. Procédé selon la revendication 5, caractérisé en ce que les résidus alanyle sont en partie les stéréo-isomères de la série D et en partie ceux de la série L.

7. Procédé selon la revendication 5 ou la revendication 6, caractérisé en ce que le rapport du nombre de résidus aminoacides de la chaîne principale par rapport à celui des ramifications est de 1/1 à 1/30, de préférence de 1/10 à 1/30.

8. Procédé selon l'une quelconque des revendications 1 à 7, caractérisé en ce que, dans le composé de muramyl-peptide, X est un résidu D-alanyle ou D-séryle.

9. Procédé selon la revendication 8, caractérisé en ce que, dans le composé de muramyl-peptide, Y est $-NH_2$, $-OCH_3$, $-OC_4H_9$ ou $OC_{10}H_{21}$.

10. Procédé selon l'une quelconque des revendications 1 à 9, caractérisé en ce que, dans le composé de muramyl-peptide, Z comprend 1 ou 2 résidus aminoacides choisis parmi ceux correspondant à l'alanine, la glycine, la valine, la leucine et l'isoleucine.

11. Procédé selon l'une quelconque des revendications 1 à 10, caractérisé en ce que, dans le composé de muramyl-peptide, R est $-CH_3$.

12. Procédé selon l'une quelconque des revendications 1 à 11, caractérisé en ce que, dans le composé de muramyl-peptide, $R_1$, $R_4$ et $R_6$ sont l'hydrogène et $R_2$ est $-CH_3$.

13. Procédé selon l'une quelconque des revendications 1 à 8, caractérisé en ce que, dans le composé de muramyl-peptide, les motifs muramyle sont des N-acétyl-muramyl-D-alanyl-D-isoglutaminyle.

**Patentansprüche für die Vertragsstaaten: BE, CH, DE, FR, GB, IT, LU, NL, SE**

1. Produkt mit einer Polymerstruktur von peptidischer Natur, umfassend eine aus Aminoacylgliedern gebildete Hauptkette, die vorzugsweise wenigstens einer der folgenden Aminosäuren entsprechen, und zwar vorzugsweise der L-Reihe: $\alpha,\gamma$-Diaminobuttersäure, Ornithin, Lysin und Homolysin, und, vorzugsweise von dieser Hauptkette abzweigend, Ketten oder Verzweigungen von peptidischer Natur, deren Glieder vorzugsweise wenigstens einem der Reste der folgenden Aminosäuren entsprechen: Alanin, Glycin, $\alpha$-Aminobuttersäure, Valin, Leucin, Isoleucin und Prolin, wobei die Aminofunktionen dieser Polymerstruktur (die nicht in diese eingebunden sind) wenigstens teilweise in kovalenter Weise an Muramyl-Peptidgruppen (oder deren Homologe oder Derivate) gebunden sind, wovon jede Gruppe eine Peptidgruppe enthält, deren an die Muramylgruppe gebundener Aminosäurerest ein Stereoisomer aus der D-Reihe ist und deren zweiter Aminorest von der D-Glutaminsäure abgeleitet ist.

2. Produkt nach Anspruch 1, gekennzeichnet durch die Formel

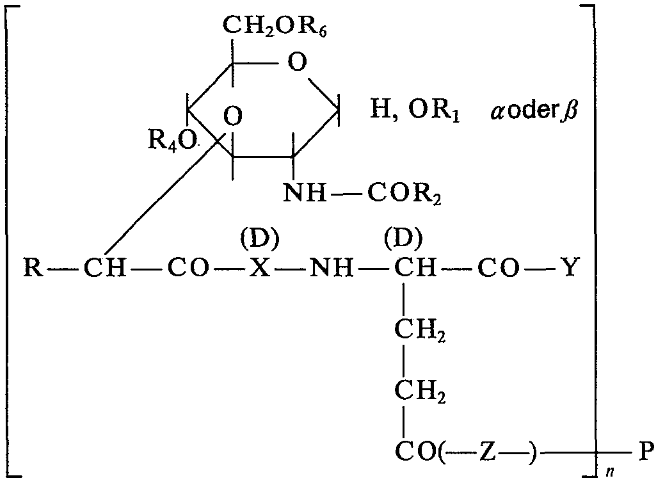

(I)

worin P die vorstehend genannte, gegebenenfalls verzweigte Polymerstruktur darstellt, wie sie vorstehend definiert ist, und den Substituenten R, $R_1$, $R_2$, $R_4$, $R_6$, X, Y und Z die folgenden Bedeutungen zukommen:

- R ist ein Wasserstoffatom oder eine Alkylgruppe mit 1 bis 4 Kohlenstoffatomen,
- $R_1$ ist ein Wasserstoffatom oder eine Alkylgruppe mit höchstens 4 Kohlenstoffatomen oder eine einfache oder substituierte Aryl- oder Alkylarylgruppe mit höchstens 10 Kohlenstoffatomen,
- $R_2$ ist ein Wasserstoffatom oder eine gegebenenfalls substituierte Alkyl-, Aryl- oder Alkylarylgruppe mit höchstens 22 Kohlenstoffatomen,
- $R_4$ ist ein Wasserstoffatom oder ein höchstens 4 Kohlenstoffatome aufweisender Acylrest,
- $R_6$ ist ein Wasserstoffatom oder eine gesättigte oder ungesättigte, gegebenenfalls verzweigte Acylgruppe mit 1 bis 90 Kohlenstoffatomen, die überdies funktionelle Gruppen aufweisen kann: Hydroxyl, Carboxyl, Amino, Cyclopropan, Methoxyl,
- X ist ein Aminoacyl der D-Reihe aus der Gruppe: Alanyl, Arginyl, Asparagyl, Aspartyl, Cysteinyl, Glutaminyl, Glutamyl, Histidyl, Hydroxyprolyl, Isoleucyl, Leucyl, Lysyl, Methionyl, Ornithyl, Phenylalanyl, Prolyl, Seryl, Threonyl, Tryptophanyl, Tyrosyl und Valyl,
- Y ist $-OH$, ein 1 bis 10 Kohlenstoffatome aufweisender Alkoxyrest oder eine $NH_2$-Gruppe, wobei die Wasserstoffatome der Aminogruppe durch Alkylreste mit 1 bis 10 Kohlenstoffatomen substituiert sein können,
- Z das in der Formel vorliegen kann oder auch nicht, stellt 1 bis 3 gleiche oder verschiedene Reste von L- oder D-Aminosäuren aus der für X angegebenen Gruppe oder Glycyl dar, und
- n ist höchstens gleich der Anzahl der Aminofunktionen, die die Polymerstruktur P aufweist.

3. Produkt nach Anspruch 1 oder 2, dadurch gekennzeichnet, daß es eine mittlere Molekularmasse zwischen 50 000 und 250 000 aufweist und daß es metabolisierbar ist.

4. Produkt nach einem beliebigen der vorstehenden Ansprüche, dadurch gekennzeichnet, daß die Hauptkette der vorgenannten Polymerstruktur zumindest teilweise aus L-Lysylresten gebildet ist.

5. Produkt nach Anspruch 4, dadurch gekennzeichnet, daß die Verzweigungen von der vorgenannten Polymerstruktur aus Alanylresten gebildet sind.

6. Produkt nach Anspruch 5, dadurch gekennzeichnet, daß die Alanylreste zum Teil die Stereoisomeren der D-Reihe und zum Teil jene der L-Reihe sind.

7. Produkt nach Anspruch 5 oder Anspruch 6, dadurch gekennzeichnet, daß das Verhältnis der Anzahl der Aminosäurereste der Hauptkette zu jener der Verzweigungen von 1 : 1 bis 1 : 30, vorzugsweise von 1 : 10 bis 1 : 30 beträgt.

8. Produkt nach einem beliebigen der Ansprüche 1 bis 7, dadurch gekennzeichnet, daß X ein D-Alanyl- oder D-Serylrest ist.

9. Produkt nach Anspruch 8, dadurch gekennzeichnet, daß Y für $-NH_2$, $-OCH_3$, $-OC_4H_9$ oder $OC_{10}H_{21}$ steht.

10. Produkt nach einem beliebigen der Ansprüche 1 bis 9, dadurch gekennzeichnet, daß Z ein oder zwei Aminosäurereste, ausgewählt unter den dem Alanin, Glycin, Valin, Leucin und Isoleucin entsprechenden Resten, enthält.

11. Produkt nach einem beliebigen der Ansprüche 1 bis 10, dadurch gekennzeichnet, daß R für $-CH_3$ steht.

12. Produkt nach einem beliebigen der Ansprüche 1 bis 11, dadurch gekennzeichnet, daß $R_1$, $R_4$ und $R_6$ für Wasserstoff stehen und $R_2$ $-CH_3$ darstellt.

13. Produkt nach einem beliebigen der Ansprüche 1 bis 8, dadurch gekennzeichnet, daß die Muramyl-Muster N-Acetyl-muramyl-D-alanyl-D-isoglutaminyl-Gruppen sind.

14. Pharmazeutische Zusammensetzung, dadurch gekennzeichnet, daß sie eine Dosis wenigstens eines Produktes nach einem beliebigen der Ansprüche 1 bis 13 im Gemisch mit pharmazeutisch annehmbaren Trägern oder Excipienten enthält.

15. Pharmazeutische Zusammensetzung nach Anspruch 14, bestehend aus einer Lösung oder Suspension eines oder mehrerer Produkte in einem injizierbaren Milieu.

16. Pharmazeutische Zusammensetzung nach Anspruch 15, dadurch gekennzeichnet, daß das injizierbare Milieu aus einer wässerigen Lösung für Injektionszwecke besteht, insbesondere aus einer isotonischen und sterilen Salz- oder Glukoselösung.

17. Pharmazeutische Zusammensetzung nach Anspruch 14, dadurch gekennzeichnet, daß das Produkt im Gemisch mit Excipienten vorliegt, die für die orale Verabreichung, für ein Aufbringen auf die Augenschleimhäute, die Atemwege oder die vaginalen Schleimhäute bzw. für eine rektale Verabreichung angepaßt sind.

18. Pharmazeutische Zusammensetzung nach Anspruch 14, dadurch gekennzeichnet, daß sie in Form von Liposomen vorliegt.

19. Pharmazeutische Zusammensetzung nach Anspruch 14, dadurch gekennzeichnet, daß für eine Antiinfektionsbehandlung auf parenteralem Wege die Dosiseinheiten 10 bis 1000 µg des vorgenannten Produktes enthalten.

20. Pharmazeutische Zusammensetzung nach Anspruch 14, dadurch gekennzeichnet, daß für eine Antiinfektionsbehandlung bei oraler Verabreichung die Dosiseinheiten 200 bis 20 000 µg des vorgenannten Produktes enthalten.

**Patentansprüche für den Vertragsstaat: AT**

1. Verfahren zur Herstellung eines Produktes mit einer Polymerstruktur von peptidischer Natur, umfassend eine aus Aminoacylgruppen gebildete Hauptkette und, im gegebenen Falle, von dieser Hauptkette abzweigende Ketten oder Verzweigungen von gleichfalls peptidischer Natur, wobei die genannte peptidische Struktur überdies Aminfunktionen aufweist, die nicht in die genannte Polymerstruktur eingebunden sind, jedoch mindestens teilweise in kovalenter Weise an Muramyl-Peptidgruppen (oder deren Homologe oder Derivate) gebunden sind, wovon jede Gruppe eine Peptidgruppe enthält, deren an die Muramylgruppe gebundene Aminosäurerest ein Stereoisomer aus der D-Reihe ist und deren zweiter Aminorest von der D-Glutaminsäure abgeleitet ist, dadurch gekennzeichnet, daß man unter auf dem Gebiet der Peptidsynthesen bekannten Bedingungen eine Kupplung kovalenter Art zwischen den nachfolgenden Komponenten bewirkt:

- freien Aminfunktionen einer Polymerstruktur von peptidischer Natur, umfassend eine aus Aminoacyleinheiten gebildete Hauptkette, die vorzugsweise wenigstens einer der folgenden Aminosäuren entsprechen: α,γ-Diamino-buttersäure, Ornithin, Lysin und Homolysin, und, in gegebenen Falle, mit Verzweigungen von gleichfalls peptidischer Natur, die von der vorgenannten Hauptkette abzweigen und aus Aminoacylresten bestehen, die vorzugsweise wenigstens einer der folgenden Aminosäuren entsprechen: Alanin, Glycin, α-Amino-buttersäure, Valin, Leucin, Isoleucin und Prolin, einerseits, und
- einem aktivierten Ester, insbesondere einer Verbindung vom Typus Muramyl-Peptid mit der durch die Formel

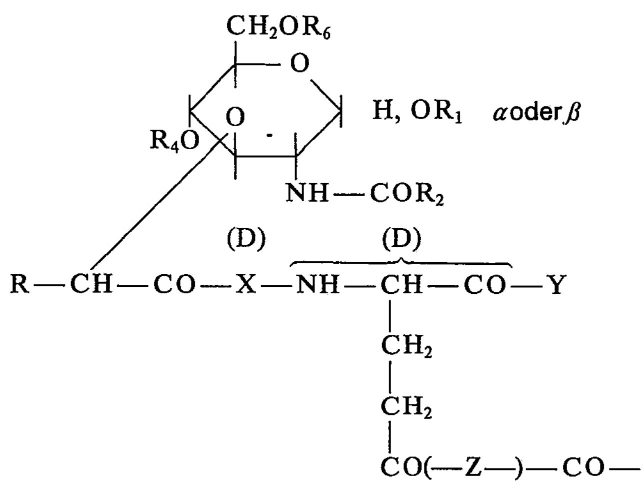

(II)

definierten Struktur, anderseits, worin:

- R ein Wasserstoffatom oder eine Alkylgruppe mit 1 bis 4 Kohlenstoffatomen ist,

- $R_1$ ein Wasserstoffatom oder eine Alkylgruppe bis höchstens 4 Kohlenstoffatomen oder eine einfache oder substituierte Aryl- oder Alkylarylgruppe mit höchstens 10 Kohlenstoffatomen ist,
- $R_2$ ein Wasserstoffatom oder eine gegebenenfalls substituierte Alkyl-, Aryl- oder Alkylarylgruppe mit höchstens 22 Kohlenstoffatomen ist,
- $R_4$ ein Wasserstoffatom oder ein höchstens 4 Kohlenstoffatome aufweisender Acylrest ist,
- $R_6$ ein Wasserstoffatom oder eine gesättigte oder ungesättigte, gegebenenfalls verzweigte Acylgruppe mit 1 bis 90 Kohlenstoffatomen ist, die überdies funktionelle Gruppen aufweisen kann: Hydroxyl, Carboxyl, Amino, Cyclopropan, Methoxyl,
- X ein Aminoacyl der D-Reihe aus der Gruppe: Alanyl, Arginyl, Asparagyl, Aspartyl, Cysteinyl, Glutaminyl, Glutamyl, Histidyl, Hydroxyprolyl, Isoleucyl, Leucyl, Lysyl, Methionyl, Ornithyl, Phenylalanyl, Prolyl, Seryl, Threonyl, Tryptophanyl, Tyrosyl und Valyl ist,
- Y für $-OH$ steht oder ein 1 bis 10 Kohlenstoffatome aufweisender Alkylrest oder eine $NH_2$-Gruppe ist, wobei die Wasserstoffatome der Aminogruppe durch Alkylreste mit 1 bis 10 Kohlenstoffatomen substituiert sein können,
- Z das in der Formel vorliegen kann oder auch nicht, 1 bis 3 gleiche oder verschiedene Reste von L- oder D-Aminosäuren aus der für X angegebenen Gruppe oder Glycyl darstellt.

2. Verfahren nach Anspruch 1, dadurch gekennzeichnet, daß der aktivierte Ester der Verbindung vom Muramyl-Peptid-Typus durch Einwirkung eines wasserlöslichen Carbodiimids und eines Alkohols auf eine entsprechende Muramyl-Peptid-Verbindung erhalten worden ist und daß die Reaktion in einer wässerigen Lösung bei einem pH-Wert von 8 bis 9 ausgeführt wird.

3. Verfahren nach Anspruch 1 oder Anspruch 2, dadurch gekennzeichnet, daß die Polymerstruktur metabolisierbar ist und daß das erhaltene Endprodukt eine mittlere Molekularmasse zwischen 50 000 und 250 000 aufweist.

4. Verfahren nach einem beliebigen der Ansprüche 1 bis 3, dadurch gekennzeichnet, daß die Hauptkette der vorgenannten Polymerstruktur zumindest teilweise aus L-Lysylresten gebildet ist.

5. Verfahren nach Anspruch 4, dadurch gekennzeichnet, daß die Verzweigungen von der vorgenannten Polymerstruktur aus Alanylresten gebildet sind.

6. Verfahren nach Anspruch 5, dadurch gekennzeichnet, daß die Alanylreste zum Teil die Stereoisomeren der D-Reihe und zum Teil jene der L-Reihe sind.

7. Verfahren nach Anspruch 5 oder Anspruch 6, dadurch gekennzeichnet, daß das Verhältnis der Anzahl der Aminosäurereste in der Hauptkette zu jener der Verzweigungen von 1 : 1 bis 1 : 30, vorzugsweise von 1 : 10 bis 1 : 30 beträgt.

8. Verfahren nach einem beliebigen der Ansprüche 1 bis 7, dadurch gekennzeichnet, daß in der Muramyl-Peptid-Verbindung X ein D-Alanyl- oder D-Serylrest ist.

9. Verfahren nach Anspruch 8, dadurch gekennzeichnet, daß in der Muramyl-Peptid-Verbindung Y für $-NH_2$, $-OCH_3$, $-OC_4H_9$ oder $OC_{10}H_{21}$ steht.

10. Verfahren nach einem beliebigen der Ansprüche 1 bis 9, dadurch gekennzeichnet, daß in der Muramyl-Peptid-Verbindung Z ein oder zwei Aminosäurereste, ausgewählt unter den dem Alanin, Glycin, Valin, Leucin und Isoleucin entsprechenden Resten, enthält.

11. Verfahren nach einem beliebigen der Ansprüche 1 bis 10, dadurch gekennzeichnet, daß in der Muramyl-Peptid-Verbindung R für $-CH_3$ steht.

12. Verfahren nach einem beliebigen der Ansprüche 1 bis 11, dadurch gekennzeichnet, daß in der Muramyl-Peptid-Verbindung $R_1$, $R_4$ und $R_6$ für Wasserstoff stehen und $R_2$ $-CH_3$ darstellt.

13. Verfahren nach einem beliebigen der Ansprüche 1 bis 8, dadurch gekennzeichnet, daß in der Muramyl-Peptid-Verbindung die Muramyl-Muster N-Acetylmuramyl-D-alanyl-D-isoglutaminyl sind.

**Claims for the Contracting States: BE, CH, DE, FR, GB, IT, LU, NL, SE**

1. Product comprising a polymeric structure of peptidic nature comprising a main chain, formed of aminoacyl units corresponding preferably to one at least of the following aminoacids, preferably of the L series: $\alpha,\gamma$-diamino-butyric acid, ornithine, lysine and homo-lysine and, preferably, branched on this main chain, chains or branches of peptidic nature the units of which correspond preferably to at least one of the residues of the following aminoacids: alanine, glycine, $\alpha$-aminobutyric acid, valine, leucine, isoleucine and proline, the amine functions of this polymeric structure (not engaged in this one) being at least in part covalently linked to muramyl peptid groups (or of their homologs or derivatives) each of these ones comprising a peptidic group in which the aminoacid residue which is linked to the muramyl group is a stereoisomer of the D series and the second amine residue derives from D-glutamic acid.

2. Product according to claim 1, characterized by the following formula:

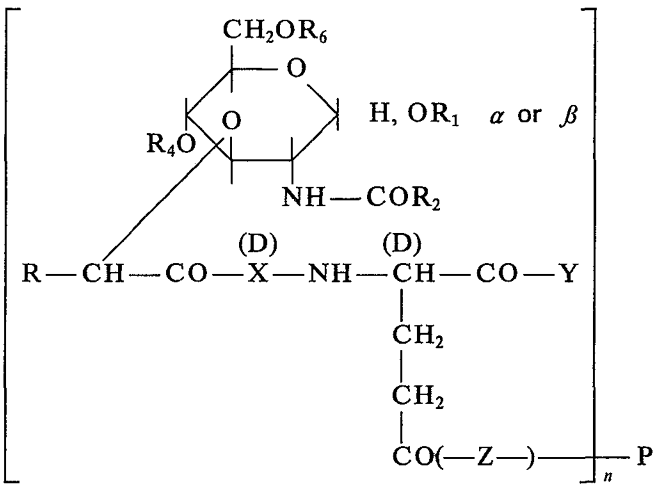

(I)

in which P represents the above said polymeric structure, possibly branched, such as above defined, and the R, $R_1$, $R_2$, $R_4$, $R_6$, X, Y and Z substituents have the following meanings:

- R is either a hydrogen atom, or an alkyl group having from 1 to 4 carbon atoms;
- $R_1$ is a hydrogen atom, or an alkyl group having at the most 4 carbon atoms, or an aryl group or a simple or substituted alkylaryl group containing at the most 10 carbon atoms;
- $R_2$ is a hydrogen atom or an alkyl group, aryl or alkylaryl group, possibly substituted and comprising at the most 22 carbon atoms;
- $R_4$ is a hydrogen atom or an acyl radical comprising at the most 4 carbon atoms;
- $R_6$ is a hydrogen atom, a saturated or unsaturated acyl group, possibly branched, containing from 1 to 90 carbon atoms and being able, moreover, to bear functional groups: hydroxyl, carboxyl, amino, cyclopropane, methoxyl;
- X is an aminoacyl group of the (D) series of the group comprising: alanyl, arginyl, asparagyl, aspartyl, cysteinyl, glutaminyl, glutamyl, histidyl, hydroxyprolyl, isoleucyl, leucyl, lysyl, methionyl, ornithyl, phenylalanyl, prolyl, seryl, threonyl, tryptophanyl, tyrosyl and valyl;
- Y is either OH or an alcoxy radical comprising from 1 to 10 carbon atoms, or a $-NH_2$ group, the hydrogen atoms of the amino group being able to be substituted by alkyl radicals comprising from 1 to 10 carbon atoms;
- Z can be present or not in the formula and represents from 1 to 3 L or D aminoacyl residues, identical or different from the group of those indicated for X or glycyl; and
- n is at the most equal to the number of the amine functions which are carried by the polymeric structure of P.

3. Product according to claim 1 or 2, characterized by the fact that it presents an average molecular weight comprised between 50 000 and 250 000, and it can be metabolized.

4. Product according to anyone of the preceding claims, characterized, by the fact that the main chain of the above polymeric structure is formed, at least in part, of L-lysyl residues.

5. Product according to claim 4, characterized by the fact that the branches of the above polymeric structure are constituted by alanyl residues.

6. Product according to claim 5, characterized by the fact that the alanyl residues are in part the stereo-isomers of the D series and in part those of the L series.

7. Product according to claim 5 or 6, characterized by the fact that the ratio of the number of aminoacyl residues of the main chain with respect to the one of the branches is from 1/1 to 1/30, preferably from 1/10 to 1/30.

8. Product according to anyone of claims 1 to 7, characterized by the fact that X ist a D-alanyl or D-seryl residue.

9. Product according to claim 8, characterized by the fact that Y is $-NH_2$, $-OCH_3$, $-OC_4H_9$ or $-OC_{10}H_{21}$.

10. Product according to anyone of claims 1 to 9, characterized by the fact that Z comprises 1 or 2 aminoacid residues selected among those corresponding to alanine, glycine, valine, leucine and isoleucine.

11. Product according to anyone of claims 1 to 10, characterized by the fact that R is $-CH_3$.

12. Product according to anyone of claims 1 to 11, characterized by the fact that $R_1$, $R_4$ and $R_6$ are hydrogen atoms and $R_2$ is $-CH_3$.

13. Product according to anyone of claims 1 to 8, characterized by the fact that the muramyl moieties are N-acetyl-muramyl-D-alanyl-D-isoglutaminyl.

14. Pharmaceutical composition, characterized by the fact that is contains an amount of at least one

product according to anyone of claims 1 to 13, in association with pharmaceutically acceptable vehicles or excipients.

15. Pharmaceutical composition according to claim 14, constituted by a solution or a suspension of the product(s) in an injectable medium.

16. Pharmaceutical composition according to claim 15, characterized by the fact that the injectable medium is constituted by an aqueous solution for injection, particularly an isotonic and sterile saline or glucose solution.

17. Pharmaceutical composition according to claim 14, characterized by the fact that the product is associated with excipients which are respectively adapted for the oral administration, for the application on to oculer mucous membranes, respiratory or vaginal tracts or for rectal administration.

18. Pharmaceutical composition according to claim 14, characterized by the fact that it is presented in the form of liposome.

19. Pharmaceutical composition according to claim 14, characterized by the fact that, for an antiinfections treatment and a parenteral administration, the unitary doses contain from 10 to 1000 μg of the above mentioned product.

20. Pharmaceutical composition according to claim 14, characterized by the fact that, for an antiinfections treatment and an oral administration, the unitary doses contain from 200 to 20 000 μg of the above mentioned product.

**Claims for the Contracting State: AT**

1. Process for preparing a product comprising a polymeric structure of peptidic nature containing a main chain formed from aminoacyl units and, possibly, branched on this main chain, chains or branches also of peptidic nature, said peptidic structure comprising moreover amine functions which are not engaged in said polymer structure, but at least in part covalently linked to muramyl peptide group (or of their homologs or derivatives) each of them comprising a peptidic group in which the aminoacid residue linked to the muramyl group is a stero-isomer of the D series, and the second amine residue derives from D-glutamic acid, characterized by the fact that a coupling is carried out covalently under known conditions in the filed of peptidic synthesis between:

- on the one hand, free amine functions of a polymer structure of a peptidic nature comprising a main chain formed from aminoacyl units corresponding preferably to at least one of the folowing aminoacids: α,γ-diamino-butyric acid, ornithine, lysine and homo-lysine, and possibly, branches themselves of peptidic nature, branched on the above said main chain, and constituted by corresponding aminoacyl residues, preferably, to at least of the following aminoacids: alanine, glycine, α-amino-butyric acid, valine, leucine, isoleucine and proline and;
- on the other hand, an activated ester, particularly a compound of the muramyl peptide type presenting the structure defined by the formula:

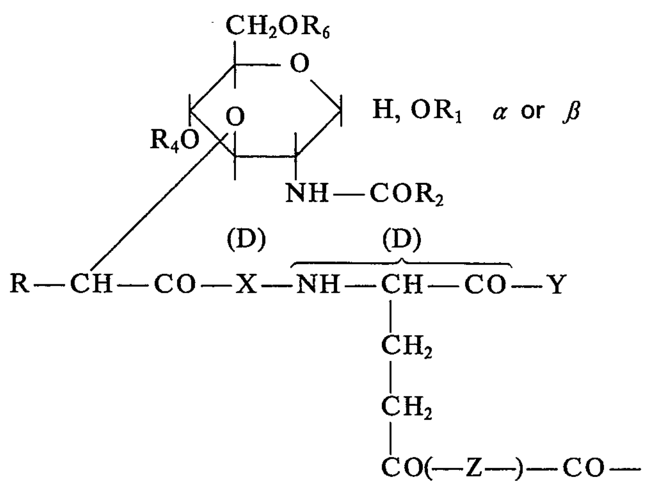

(II)

in which:

- R is either a hydrogen atom, or an alkyl group having from 1 to 4 carbon atoms;
- $R_1$ is a hydrogen atom, or an alkyl group at the most 4 carbon atoms, or an aryl group or a simple or substituted alkylaryl group containing at the most 10 carbon atoms;
- $R_2$ is a hydrogen atom or an alkyl group, aryl or alkylaryl group, possibly substituted and comprising at the most 22 carbon atoms;
- $R_4$ is a hydrogen atom or an acyl radical comprising at the most 4 carbon atoms;

– $R_6$ is a hydrogen atom, a saturated or unsaturated acyl group, possibly branched, containing from 1 to 90 carbon atoms and being able, moreover, to bear functional groups: hydroxyl, carboxyl, amino, cyclopropane, methoxyl;
– X is an aminoacyl group of the (D) series of the group comprising: alanyl, arginyl, asparagyl, aspartyl, cysteinyl, glutaminyl, glutamyl, histidyl, hydroxyprolyl, isoleucyl, leucyl, lysyl, methionyl, ornithyl, phenylalanyl, prolyl, seryl, threonyl, tryptophanyl, tyrosyl and valyl;
– Y is either $-OH$ or an alcoxy radical comprising from 1 to 10 carbon atoms, or a $-NH_2$ group, the hydrogen atoms of the amino group being able to be substituted by alkyl radicals comprising from 1 to 10 carbon atoms;
– Z can be present or not in the formula and represents from 1 to 3 L or D aminoacyl residues, identical or different from the group of those indicated for X or glycyl.

2. Process according to claim 1, characterized by the fact that the activated ester of the compound of the muramyl peptide type has been obtained by action on a compound of a corresponding muramyl peptide of a hydrosoluble carbodiimide and an alcohol and that the reaction is carried out within an aqueous solution at pH 8–9.

3. Process according to claim 1 or to claim 2, characterized by the fact that the polymer structure can be metabolized and by the fact that the final product which is obtained presents an average molecular weight comprised between 50 000 and 250 000.

4. Process according to anyone of claims 1 to 3, characterized by the fact that the main chain of the said polymer structure is formed, at least in part, of L-lysyl residues.

5. Process according to claim 4, characterized by the fact that the branches of the above said polymer structure are constituted by alanyl residues.

6. Process according to claim 5, characterized by the fact that the alanyl residues are in part the stereo-isomers of the D series and in part those of the L series.

7. Process according to claim 5 or to claim 6, characterized by the fact that the ratio of the number of the aminoacyl residues of the main chain with respect to the one of the branches is of 1/1 to 1/30, preferably of 1/10 to 1/30.

8. Process according to anyone of claims 1 to 7, characterized by the fact that in the muramyl peptide compound, X is a D-alanyl or D-seryl residue.

9. Process according to claim 8, characterized by the fact that in the muramyl peptide compound, Y is $-NH_2$, $-OCH_3$, $-OC_4H_9$ or $-OC_{10}H_{21}$.

10. Process according to anyone of claims 1 to 9, characterized by the fact that in the muramyl peptide compound, Z comprises 1 or 2 aminoacyl residues chosen from among those corresponding to alanine, glycine, valine, leucine or isoleucine.

11. Process according to anyone of claims 1 to 10, characterized by the fact that in the compound of muramyl peptide, R is $-CH_3$.

12. Process according to anyone of claims 1 to 11, characterized by the fact that in the compound of muramyl peptide, $R_1$, $R_4$ and $R_6$ are hydrogen atoms and $R_2$ is $-CH_3$.

13. Process according to anyone of claims 1 to 8, characterized by the fact that in the compound of muramyl peptide, the muramyl moieties are N-acetyl-muramyl-D-alanyl-D-isoglutaminyl.